(19) Europäisches Patentamt
European Patent Office
Office européen des brevets

(11) Veröffentlichungsnummer: **0 114 051**
**B1**

# (12) EUROPÄISCHE PATENTSCHRIFT

(45) Veröffentlichungstag der Patentschrift: **18.03.87**

(21) Anmeldenummer: **84100121.7**

(22) Anmeldetag: **09.01.84**

(51) Int. Cl.⁴: **A 61 K 7/00**, A 61 K 31/235, A 61 K 31/24, C 07 C 69/92, C 07 C 93/20

## (54) Verwendung von sebosuppressiven kosmetischen Mitteln, enthaltend Alkoxybenzoesäureester.

(30) Priorität: **17.01.83 DE 3301313**

(43) Veröffentlichungstag der Anmeldung: **25.07.84 Patentblatt 84/30**

(45) Bekanntmachung des Hinweises auf die Patenterteilung: **18.03.87 Patentblatt 87/12**

(84) Benannte Vertragsstaaten: **AT BE CH DE FR GB IT LI LU NL SE**

(56) Entgegenhaltungen:
**EP-A-0 011 845**
**DE-A-3 047 106**
**DE-A-3 121 064**
**DE-C-658 389**

**P.H. LIST, L. HÖRHAMMER "Hagers Handbuch der Pharmazeutischen Praxis" 4. Ausgabe, 2. Band, 1969, SPRINGER VERLAG, Berlin-Heidelberg-New York, Localanästhesica Seiten 275-304**
**BEILSTEINS HANDBUCH DER ORGANISCHEN CHEMIE, 4. Auflage, 10. Band, 1.Teil, DRITTES ERGÄNZUNGSWERK, H.G. Boit, 1971, Springer Verlag, Berlin-Heidelberg-New York**
**BEILSTEINS HANDBUCH DER ORGANISCHEN CHEMIE, 4. Auflage, 10. Band, 1. Teil VIERTES ERGÄNZUNGSWERK, R. Luckenbach, 1983**
**J. Pharm. Pharmacol., Bd.6, (1954), S. 119-121**

(73) Patentinhaber: **Henkel Kommanditgesellschaft auf Aktien, Postfach 1100 Henkelstrasse 67, D-4000 Düsseldorf- Holthausen (DE)**

(72) Erfinder: **Möller, Hinrich, Dr., Schumannstrasse 11, D-4019 Monheim (DE)**
Erfinder: **Wallat, Siegfried, Dr., Marie- Curie-Strasse 9, D-4019 Monheim (DE)**

(56) Entgegenhaltungen: (Fortsetzung)
**Die Akte enthält technische Angaben, die nach dem Eingang der Anmeldung eingereicht wurden und die nicht in dieser Patentschrift enthalten sind.**

**EP 0 114 051 B1**

Anmerkung: Innerhalb von neun Monaten nach der Bekanntmachung des Hinweises auf die Erteilung des europäischen Patents im Europäischen Patentblatt kann jedermann beim Europäischen Patentamt gegen das erteilte europäische Patent Einspruch einlegen. Der Einspruch ist schriftlich einzureichen und zu begründen. Er gilt erst als eingelegt, wenn die Einspruchsgebühr entrichtet worden ist (Art. 99(1) Europäisches Patentübereinkommen).

LIBER, STOCKHOLM 1987

**Beschreibung**

Die Erfindung betrifft topische, kosmetische Mittel zur Verbesserung des fettigen und unästhetischen Aussehens der Haare und der Haut, welche bestimmte Alkoxybenzoesäureester enthalten, sowie neue Alkoxybenzoesäureester.

Die moderne Kosmetik ist bemüht, das durch übermäßig starke Absonderung der Talgdrüsen und der Kopfhaut verursachte fettige und wenig ästhetische Aussehen der Haare zu vermindern. Es ist daher vielfach versucht worden, durch geeignete Mittel die Sekretion der Talgdrüsen zu normalisieren, um dem Haar wieder sein gesundes Aussehen zu geben. Es wurden zur Bekämpfung der Seborrhoe des behaarten Kopfes kosmetische Pflegemittel mit Schwefel-, Quecksilber- oder Teerzusatz verwendet. Dabei hat sich gezeigt, daß diese bekannten antiseborrhoischen Zusätze bei längerer Anwendung häufig zu Nebenwirkungen führen, ohne daß wirklich befriedigende Ergebnisse im Hinblick auf Wirksamkeit und anwendungstechnische Eigenschaften erzielt werden konnten. Aus J. Pharm. Pharmacol., Band 6, 1954, Seiten 119-121 sind p-Alkoxybenzoesäureester als Lokalanästhetika bekannt. DE-A-31 21 064 beschreibt die Verwendung von p-Alkoxybenzoesäureestern von Alkoholen mit 1-12 C-Atomen als antiseborrhoische Zusätze zu kosmetischen Mitteln; diese weisen jedoch eine noch nicht befriedigende Wirkung auf.

Aufgabe der Erfindung ist es, ein kosmetisches Mittel bereitzustellen, welches gegenüber den bekannten Präparaten eine verstärkte Wirkung, ohne nachteilige Folgen auf den menschlichen Körper hat.

Es wurde nun überraschend gefunden, daß bestimmte Alkoxybenzoesäureester hervorragende antiseborrhoische Effekte auch bei sehr geringer Dosierung aufweisen.

Gegenstand der Erfindung ist die Verwendung von kosmetischen Mitteln, mit einem Gehalt an p-Alkoxybenzoesäureestern der allgemeinen Formel

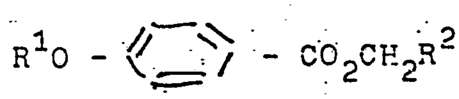

worin bedeuten

$R^1$ einen n-Alkylrest mit 6 - 20 C-Atomen

$R^2$ eine der Gruppen $CH_2OR^3$, $C_2H_4OR^3$, $CH_2OC_2H_4OR^3$, $CH_2OH$, $C_2H_4OH$, $CHCH_3OH$, $CH_2NR^3R^4$, $C_2H_4NR^3R^4$, $CO_2R^3$ oder $CH_2CO_2R^3$,

$R^3$ und $R^4$ gleiche oder verschiedene Alkylreste mit 1 - 4 C-Atomen,

zur Behandlung von seborrhoischer Haut und fetten Haaren.

Die anspruchsgemäß zu verwendenden Verbindungen können nach allgemein bekannten Verfahren hergestellt werden.

Zum Beispiel können die Alkoxyalkyl- bzw. Hydroxyalkylester aus den entsprechenden p-Alkoxybenzoesäuremethylestern durch Umesterung mit geeigneten Alkoholkomponenten mit Resten gemäß $R^2$ unter alkylischer Katalyse, wie beispielsweise Natriummethoxylat, erhalten werden. Das freiwerdende Methanol wird durch Destillieren aus dem Gleichgewicht entfernt.

Die p-Alkoxy-benzoesäuremethylester werden z. B. durch Alkylierung von p-Hydroxybenzoesäuremethylester mit Alkylhalogeniden, -sulfaten oder-sulfonaten mit Alkylresten entsprechend $R^1$ hergestellt.

Man kann auch in umgekehrter Weise zunächst die Veresterung der p-Hydroxy-benzoesäure durchführen und danach die Alkylierung vornehmen.

Ein weiterer Weg zur Herstellung der neuen Ester besteht darin, daß man die Alkalisalze, insbesondere Natriumsalze, der p-Alkoxy-benzoesäuren mit Alkylhalogeniden, -sulfaten oder -sulfonaten mit Resten gemäß $R^2$ unter Alkylierung umsetzt. Dieses Verfahren eignet sich besonders zur Herstellung der p-Alkoxybenzoesäurealkoxycarbonylalkylester, wobei entsprechende Halogenalkansäureester als Alkylierungsmittel dienen.

Schließlich kommt auch noch das direkte Veresterungsverfahren infrage, bei dem die Ester aus den entsprechenden p-Alkoxy-benzoesäuren und Alkanolen mit Resten gemäß $R^2$ durch Wasserabspaltung in Gegenwart saurer Katalysatoren gebildet werden. Bei Anwendung eines großen Alkohoüberschusses kann das Reaktionswasser im Reaktionsgemisch verbleiben, andernfalls wird es durch geeignete Maßnahmen, z. B. Destillation, aus dem Gleichgewicht entfernt.

Folgende p-Alkoxy-benzoesäuren können den beanspruchten Estern beispielsweise zugrunde liegen:

4-Hexyloxy-, 4-Octyloxy-, 4-Decyloxy- 4-Undecyloxy-,
4-Dodecyloxy-, 4-Tetradecyloxy-, 4-Hexadecyloxy-,
4-Octadecyloxy-, 4-Eicosyloxy-benzoesäure.

Die Alkoholkomponente kann beispielsweise folgende Reste (gemäß $R^2$) enthalten:

2-Hydroxyethyl, 2-, 3-Hydroxypropyl, 2-Methoxyethyl,
2-Ethoxyethyl, 3-Methoxypropyl, 2-Diethylamino-ethyl,
3-Dimethylamino-propyl, Methoxy-, Ethoxy-, Propoxycarbonylmethyl und 2-Methoxy-carbonyl-ethyl.

Die zu verwendenden Verbindungen besitzen eine ausgeprägte sebosuppressive Wirkung bei ausgezeichneter Haut,-und Schleimhautverträglichkeit. Sie lassen sich ohne Schwierigkeit in verschiedene kosmetische Zubereitungen, wie wäßrige oder alkoholische Lösungen, Öle, Suspensionen, Gele, Emulsionen, Salben oder Aerosole einarbeiten. Zur Behandlung von seborrhoischer Haut und fetten Haaren können diese in allen üblichen Applikationsformen, wie Haarwässern, Haarshampoos, Haarkuren, Haarspülungen, Hautlotionen oder Schüttelmixturen eingesetzt werden. Bevorzugt ist die Verwendung in Haarpflegemitteln. Neben der erfindungsgemäßen Wirkstoffkombination können diese kosmetischen Mittel übliche Träger- und Hilfsstoffe, wie Wasser, organische Lösungsmittel, oberflächenaktive Verbindungen, Öle, Fette, Wachse, Duftstoffe, Farbstoffe, Konservierungsmittel und dergleichen enthalten. Die neuen sebosuppressiven Mittel enthalten zweckmäßig 0,01 - 10 Gew.-%, vorzugsweise 0,05 - 5 Gew.-% der p-Alkoxy-benzoesäurederivate.

**Herstellungsbeispiele**

**A) p-Decyloxy-benzoesäure-2-methoxyethyl-ester**

**a) p-Hydroxy-benzoesäure-2-methoxyethyl-ester**

Eine Mischung aus 25,0 g p-Hydroxy-bensoesäure, 170 g Methylglykol und 1 ml Schwefelsäure (konz.) wurde 8 h auf 110 - 115 ° erwärmt und nach dem Abdestillieren des überschüssigen Methylglykols unter vermindertem Druck auf Eis/Wasser gegossen. Nach Neutralisieren mit Natriumhydrogencarbonat wurde die wäßrige Phase mehrmals mit Methylenchlorid ausgeschüttelt, die Methylenchloridlösung mit Aktivkohle behandelt, eingedampft und der Rückstand aus Toluol umkristallisiert. Es wurden 27,6 g (78 %) p-Hydroxy-benzoesäure-2-methyloxyethyl-erster vom Schmp. 90 - 93 °C erhalten.

**b) p-Decyloxy-benzoesäure-2-methoxyethyl-ester**

Die Mischung aus frisch hergestelltem Natriumethanolat (aus 2,3 g (0,1 Mol) Natrium und 100 ml Ethanol, zur Trockene gedampft), 250 ml getrocknetem Dimethylformamid und 17,7 g (0,1 Mol) Decylchorid wurde 4 h zum Sieden erhitzt und anschließend im Ölpumpenvakuum konzentriert. Der Rückstand wurde in Petrolether aufgenommen. 3mal mit Wasser ausgewaschen, die Petroletherlösung mit $Na_2SO_4$ getrocknet und eingedampft. Man erhielt so 25,5 g noch schwach mit Ausgangsprodukt verunreinigten p-Decyloxy-benzoesäure-2-methoxyethyl-ester. Reiner Ester wurde durch Säulenchromatographie ($SiO_2$, Merck/ Methylenchlorid + 2 % Methanol) erhalten.

Brechungsindex: $n_D^{20} = 1{,}4971$.

**B) p-Decyloxy-benzoesäure-2-ethoxyethyl-ester**

wurde analog A) hergestellt: $n_D^{20} = 1{,}4932$

**C) p-Decyloxy-benzoesäure-2-(2-methoxyethoxy)-ethyl-ester**

Die Mischung aus 25 g (0,09 Mol) p-Decyloxy-benzoesäuremethylester und 100 ml Diethylenglykolmonomethylether wurde mit einer Spatelspitze Natriummethanolat 6 h auf 150 °C erwärmt. Nach dem Abdestillieren des überschüssigen Diethylenglykolmonomethylethers wurde der Rückstand in Methylenchlorid aufgenommen, die Lösung mit Wasser gewaschen, mit Aktivkohle behandelt und eingedampft. Es wurden 24,5 g (72 %) noch schwach verunreinigte p-Decyloxy-benzoesäure-2-(2-methoxyethoxy)-ethyl-ester erhalten. Säulenchromatographische Reinigung ($SiO_2$, Merck/ Methylenchlorid + 5 % Methanol) ergab reinen Ester vom Brechungsindex: $n_D^{20} = 1{,}4945$.

**D) p-Decyloxy-benzoesäure-2-hydroxyethyl-ester**

wurde analog C) hergestellt. Schmp. 42 - 44 °C

**E)p-Decyloxy-benzoesäure-2-diethylaminoethyl-ester**

wurde analog C) hergestellt: $n_D^{20} = 1{,}4975$

**F) p-Decyloxy-benzoesäure-2-methoxyethyl-ester**

wurde analog A) hergestellt: $n_D^{20} = 1{,}4945$

**G) p-Decyloxy-benzoyloxyessigsäure-methyl-ester**

Die Suspension von 20 g (67 m Mol) p-Decyloxy-benzoesäure, Natriumsalz, in 100 ml Methanol wurde nach Zugabe von 11,2 g (73 m Mol) Bromessigsäuremethylester 3 h und nach weiterer Zugabe von 2 ml Bromessigsäuremethylester noch 1,5 h zum Sieden erhitzt. Nach Abdestillieren der flüchtigen Bestandteile wurde der Rückstand mit Methylenchlorid kalt extrahiert, die Lösung mit Wasser gewaschen, eingedampft, der Ölige Rückstand in Petrolether aufgenommen, die Lösung mit Aktivkohle behandelt und wieder eingedampft. Es wurden 15,8 g (68 %) p-Decyloxybenzoyloxyessigsäure-methyl-ester vom Schmp. 30 - 32 °C erhalten.

Säulenchromatographische Reinigung ($SiO_2$, Merck/ Methylenchlorid : Toluol = 7 : 3) ergab reinen Ester vom Schmp. 32,5 - 34 °C.

Die antiseborrhoische Wirkung wurde durch nachfolgend beschriebene Tierversuche näher untersucht.

Als Versuchstiere dienten männliche Wistar-Ratten mit einem Körpergewicht von 220 - 230 g zu Versuchsbeginn. Beurteilt wurde visuell der Bräunungsgrad auf dem Rücken der dort geschorenen Ratten. Die Bräunung wird durch das braune Hautoberflächenlipid der Ratten hervorgerufen. Dieser Test geht von der Beobachtung aus, daß junge weibliche Ratten sowie männliche Ratten nach dem Waschen mit Tensidlösung

bzw. einem Lipidlösungsmittel und auch männliche Ratten, die systematisch mit Östrogen behandelt wurden, nur die normale helle, rosafarbene Haut nach dem Scheren aufweisen; parallel dazu sind aus den abgeschnittenen Haaren nur noch vergleichsweise sehr geringe Lipidmengen zu extrahieren.

Zur Beurteilung der Wirksamkeit wurden die Prüfsubstanzen in alkoholischer Lösung jeweils 6 Ratten halbseitig auf das Rückenfell gepinselt. Die andere Seite wurde nur mit dem Lösungsmittel ohne Wirkstoffe behandelt.

Während der Versuchsdauer von 14 Tagen wurde an insgesamt 9 Tagen einmal appliziert. Zur weiteren Kontrolle diente eine Gruppe von 6 Ratten, die völlig unbehandelt blieben. Am Ende des Versuchs wurden die Tiere am Rücken und an den Flanken geschoren und von einem Beurteilerpanel (6 Personen) unahhängig unter Doppelblindbedingungen visuell abgemustert.

**Bewertungsmethoden:**

Als 1. Kriterium wurde bewertet, ob die Mehrheit der Beurteiler richtig die behandelte Seite erkannt haben, wobei wie folgt differenziert wurde:

| Zeichen | Anteil der Beurteiler, die eine Wirkung erkannten |
|---|---|
| ++ | 100 % |
| + | > 50 % — 100 % |
| - | ≤ 50 % |

Als 2. Kriterium wurde der Unterschied zwischen rechter und linker Seite gewertet, wobei Pro Beurteiler und Tier jeweils 1 Punkt zu vergeben war, und zwar in der Weise, daß die
dunklere Seite mit 1 Punkt
hellere Seite mit 0 Punkte und
bei Gleichheit beide Seiten mit 0,5 Punkte benotet wurden.

Signifikante Differenzen zwischen unbehandelter und behandelter Seite nach der zweiten Bewertungsmethode zeigen die lokale Wirksamkeit einer Substanz an.

Als 3. Kriterium wurden außerdem noch die Intensitätsunterschiede der Brauntöne nach folgender Skala bewertet:
3 Punkte stark braun
2 Punkte mittel braun
1 Punkt schwach braun
0 Punkte keine Braunfärbung.

Nach der dritten Bewertungsmethode werden die Punktsummendifferenzen zwischen den unhehandelten Kontrolltieren und jeweils den behandelten und unbehandelten Seiten der Versuchstiere gebildet, wobei wiederum signifikante Differenzen zwischen Kontrolltieren und der behandelten Seite der Versuchstiere die Wirkung einer Substanz deutlich machen.

Parallel dazu ist in der Regel auch ein deutlicher Unterschied zwischen der unbehandelten und der behandelten Seite der Versuchstiergruppen zu sehen. Dieser ist aber nicht immer so deutlich wie der zwischen Kontrolltieren und behandelter Seite, wofür es verschiedene Gründe geben kann, wie zum Beispiel mechanische Substanzübertragung von einer auf die andere Seite oder Lösungsmitteleinfluß.

Zur Differenzierung der Effekte gemäß Beurteilungsmethode 2 und 3 wurde das folgende Schema verwendet:

| Zeichen | Punktdifferenz |
|---|---|
| ++ | sehr groß (≥ 99,9 % Wahrscheinlichkeit) |
| + | signifikant (≥ 95 % Wahrscheinlichkeit) |
| - | (< 95 % Wahrscheinlichkeit) |

**Prozentuale Sebumreduktion**

Die Sebumreduktion errechnet sich aus der Punktedifferenz in der Weise, daß man den Quotienten aus der Punktedifferenz $\Delta$ P und der Punktezahl für die Kontrolgruppe $P_k$ bildet und den erhaltenen Wert in % angibt.
Sebumreduktion =

$$\frac{\Delta P}{P_k} \cdot 100 \; [\%]$$

Die p-Alkoxy-benzoesäureester wurden in Konzentrationen von 0,1 bzw. 0,2 und 1,0 % in Alkohol in der angegebenen Weise appliziert. Die Ergebnisse sind in der Tabelle zusammengefaßt.

**Tabelle**

Bewertung der sebosuppressiven Effekte

| Substanz | Konz. (%) | Bewertungsmethode 1 | 2 | 3 | Sebumreduktion (%) |
|---|---|---|---|---|---|
| A | 1,0 | ++ | ++ | ++ | 100 |
| | 0,1 | ++ | ++ | ++ | 69 |
| B | 1,0 | ++ | ++ | ++ | 93 |
| | 0,1 | ++ | ++ | ++ | 30 |
| C | 1,0 | ++ | ++ | ++ | 100 |
| | 0,1 | ++ | ++ | ++ | 26 |
| D | 1,0 | ++ | ++ | ++ | 98 |
| E | 1,0 | ++ | ++ | ++ | 90 |
| | 0,1 | ++ | ++ | ++ | 80 |
| F | 1,0 | ++ | ++ | ++ | 91 |
| | 0,2 | ++ | ++ | ++ | 92 |
| G | 0,5 | ++ | ++ | ++ | 82 |

**Beispiele für Rezepturen**

Nachfolgend werden für die erfindungsgemäßen topischen Mittel zur Behandlung von stark fettendem Haar und seborrhoischer Haut Rezepturen gegeben:

**1. Shampoo für fettendes Haar**

Gewichteile
Ammoniumlaurylsulfat mit 33-35%
Waschaktivsubstanz (Texapon A®) 40,0
Kokosfettsäurediethanolamid
(Comperlan KD ®) 3,0
Natriumchlorid 2,0
Natriumsulfat 2,0
Substanz nach Beispiel A 1.0
Konservierungsmittel 0,1
Parfümöl 0,1
Wasser ad 100

**2. Haarkur**

Gewichtsteile
Glycerinmono-distearat
(Tegin M®) 0,7
kationisches Tensid 2,0
Cholesterin 0,2
Sojalecithin 0,3
Emulgade A® (Gemisch von Cetylstearylalkohol mit nichtionogenen Emulgatoren) 8,0
Parfümöl 0,3
Substanz nach Beispiel D 1,0
Wasser, vollentsalzt ad 100

**3. Hautcreme**

Selbstemulgierendes Gemisch aus Mono/Diglyceriden höherer gesättigter Fettsäuren mit Kaliumstearat (Cutina KD 16®) 16,0
Cetylstearylalkohol mit ca. 12 Mol
Ethylenoxid (Eumulgin B 1® 1,0
2-Octyldodecanol 6,0
Isopropylmyristst 4,0
Glycerin 6,0
Substanz nach Beispiel F 2,0
Wasser ad 100
Bezugsquellen für die genannten Handelsprodukte
Texapon A® = Henkel KGaA
Comperlan KD® = Henkel KGaA
Emulgade A® = Henkel KGaA
Cutina KD 16® = Henkel KGaA
Eumulgin B 1® = Henkel KGaA
Tegin M® = Atlas-Chemie

**Patentansprüche**

1. Verwendung von kosmetischen Mitteln mit einem Gehalt an p-Alkoxy-benzoesäureestern der allgemeinen Formel

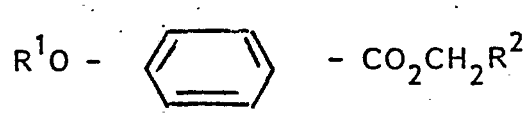

worin bedeuten
$R^1$ einen n-Alkylrest mit 6 - 20 C-Atomen
$R^2$ eine der Gruppen
$CH_2OR^3$, $C_2H_4OR^3$, $CH_2OC_2H_4OR^3$,
$CH_2OH$, $C_2H_4OH$, $CHCH_3OH$, $CH_2NR^3R^4$,
$C_2H_4NR^3R^4$, $CO_2R^4$ oder $CH_2CO_2R^3$,
$R^3$ und $R^4$ gleiche oder verschiedene Alkylreste mit 1 - 4 C-Atomen
zur Behandlung von seborrhoischer Haut und fetten Haaren.

2. Verwendung nach Anspruch 1, dadurch gekennzeichnet, daß die kosmetischen Mittel einen Gehalt von 0,05 - 5,0 Gew.-% an p-Alkoxybenzoesäureestern enthalten.

**Claims**

1. The use of cosmetic preparations containing p-alkoxybenzoic acid esters corresponding to the following general formula

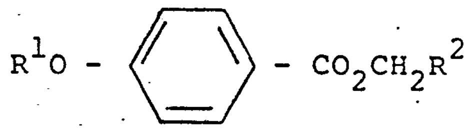

in which
$R^1$ is a $C_6$-$C_{20}$ n-alkyl group,
$R^2$ is one of the groups
$CH_2OR^3$, $C_2H_4OR^3$, $CH_2OC_2H_4OR^3$
$CH_2OH$, $C_2H_4OH$, $CHCH_3OH$, $CH_2NR^3R^4$,
$C_2H_4NR^3R^4$, $CO_2R^4$ or $CH_2CO_2R^3$ and
$R^3$ and $R^4$ may be the same or different and represent $C_1$-$C_4$ alkyl groups,
for the treatment of seborrhoeic skin and greasy hair.

2. The use claimed in Claim 1, characterized in that the cosmetic preparations contain from 0.05 to 5.0% by weight p-alkoxybenzoic acid esters.

## Revendications

1°) Utilisation de produits cosmétiques ayant une teneur en esters d'acide p-alcoxy-benzoïque de formule générale:

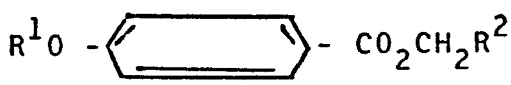

dans laquelle:
$R^1$ represente un reste n-alkyle ayant de 6 à 20 atomes de carbone;
$R^2$ représente un des groupes $CH_2OR^3$, $C_2H_4OR^3$, $CH_2OC_2H_4OR^3$,
$CH_2OH$, $C_2H_4OH$, $CHCH_3OH$, $CH_2NR^3R^4$,
$C_2H_4NR^3R^4$, $CO_2R^4$ ou $CH_2CO_2R^3$,
$R^3$ et $R^4$ étant des restes alkyle identiques ou différents ayant de 1 à 4 atomes de carbone;
pour le traitement de la peau séborrhéique et de cheveux gras.

2°) Utilisation selon la revendication 1, caractérisée en ce que les produits cosmétiques ont une teneur en esters d'acide p-alcoxybenzoïque allant de 0,05 à 5,0 % en poids.